Europäisches Patentamt

European Patent Office

Office européen des brevets

(19)

(11) Publication number: **0 333 678**
A2

(12)

# EUROPEAN PATENT APPLICATION

(21) Application number: 89850005.3

(22) Date of filing: 02.01.89

(51) Int. Cl.⁴: **A 61 K 31/08**

(30) Priority: **21.01.88 SE 8800182**

(43) Date of publication of application:
**20.09.89 Bulletin 89/38**

(84) Designated Contracting States:
**AT BE CH DE ES FR GB GR IT LI LU NL SE**

(71) Applicant: **HÄLSOPRODUKTER LARS KÄRNERUD AB**
**Box 145**
**S-57022 Forserum (SE)**

(72) Inventor: **Brohult, Sven**
**Gliavägen 97**
**S-161 52 Bromma (SE)**

**Brohult, Astrid**
**Gliavägen 97**
**S-161 Bromma (SE)**

(74) Representative: **Arwidi, Bengt**
**AHLPATENT AB Hemstigen 21**
**S-552 66 Jönköping (SE)**

The title of the invention has been amended (Guidelines for Examination in the EPO, A-III, 7.3).

(54) **Use of glycerol ethers in the treatment of allergic diseases.**

(57) This invention relates to use of at least one glycerol ether for a pharmaceutical preparation for treatment of allergic diseases, especiall asthma. The pharmaceutical is administered orally using capsules. The active substance comprises a mixture of glycerol ethers and is produced from shark liver.

EP 0 333 678 A2

## Description

### Use of glycerol ether

This invention relates to use of at least one glycerol ether for a pharmaceutical preparation. The pharmaceutical preparation is for treatment of allergic diseases, especially asthma.

Glycerol ethers (previously also called alcoxyglycerols) have shown several important medical effects. Oral administration of glycerol ethers by radiation therapy reduces the number of harmful radiation effects; leucopenia and trombocytopenia is partly or fully prevented. By certain tumor diseases a lower mortality is obtained in a group which has been given glycerol ethers as profylactic treatment as compared to a group which was not given these substances. Experiments both with humans and animals have shown that the glycerol ethers improve the bodily immunity defense mechanism.

In a human body glycerol ethers are found mainly in bone marrow, liver, mother's milk and placenta. The glycerol ethers are found in larger quantities mainly in shark liver oil.

The glycerol ethers have the following general formula:

$CH_2$ . OH
CH . OH
$CH_2$ . O . R,

wherein R represents a longchained, aliphatic hydrocarbon chain. Naturally occurring glycerol ethers are mixtures with varying number of carbon atoms in the side chain. Compounds having 16 or 18 carbon atoms make up most the quantities, but the nubmer of carbon atoms varies mainly with the interval of 14 - 24. E.g. chimmyl alcohol and batyl alcohol are saturated glycerol ethers having 16 and 18 carbon atoms respectively in the side chain. Selachyl alcohol is an unsaturated glycerol ether with 18 carbon atoms. There also are found methoxysubstituted glycerol ethers, i.e. compounds wherein one hydrogen atom, mostly on carbon atom 2, has been replaced by a methoxy group ($-OCH_3$).

In the human body like in shark liver oil the glycerol ethers are present as di-esters of fatty acids. They differ from ordinary fat (triglycerid, i.e. a tri-ester of fatty acids) only at one point in the molecule, namely that one of the three ester bonds is replaced by an ether bond.

Diseases, which appear when a person is oversensitive to a foreign substance, are named allergies. These have increased considerably during the latest 20-30 years, perhaps mainly due to the increase of foreign substances in the environment. More than 1 million Swedes have one kind or another of allergy.

It has now unexpectedly been found, that the discomforts by several different allergies are greatly reduced by administration of glycerol ethers. Some persons, being over-sensitive to certain foods (unsaturated fats, fish, shell-fish) have become almost entirely free from the allergic reactions. Especially remarkable is the result by treatment of asthma. After 3 to 4 months the discomforts start to disappear. In some cases a complete recovery has been observed after 6 months. Especially one case as attracted much interest. That person had had severe asthma for 15 years and was totally dependent of various asthma medicals (betaprostimulators, broncodilaters, corticosteroids). After $3\frac{1}{2}$ months the discomforts started to disappear. The asthma medicals were successively reduced during 3 months further and were thereafter completely removed. That person seems now to have fully recovered.

The glycerol ethers have been administered orally using capsules, which are available under the trademark ECOMER. Each capsule contains 0.05 g of the active substance, which comprises a mixture of glycerol ethers and is produced from shark liver.

## Claims

1) Use of at least one glycerol ether for making a pharmaceutical preparation for treatment of allergic diseases, especially asthma.

2) Use according to claim 1, **characterized** in that the mixture of glycerol ethers being present in shark liver oil is used.

3) Use according to claim 1, **characterized** in that one or more glycerol ethers with a straight side chain is used.

4) Use according to claim 1, **characterized** in that one or more methoxy-substituted glycerol ethers is used.

5) Use according to claim 1, **characterized** in that a mixture of glycerol ethers having straight side chains and methoxy-substituted glycerol ethers is used.